# EUROPÄISCHE PATENTSCHRIFT

(11) **EP 2 275 530 B1**
(45) Veröffentlichungstag und Bekanntmachung des Hinweises auf die Patenterteilung: **07.08.2019**
(21) Anmeldenummer: 10176622.8
(22) Anmeldetag: 07.11.2003
(51) Int. Cl.: C12N 5/00, C12P 21/02

(54) **Verfahren zur Kultivierung von Zellen zur Produktion von Substanzen**
Method for cultivating cells for the production of compounds
Procédé pour cultiver des cellules pour la production de substances

(30) Priorität: 27.11.2002 DE 10255508
(43) Veröffentlichungstag der Anmeldung: 19.01.2011
(62) Teilanmeldung aus: 10152393.4
(73) Patentinhaber: F. Hoffmann-La Roche AG, 4070 Basel (CH)
(72) Erfinder: Essers, Ruth, 51063 Koeln (DE); Gaetgens, Jochen, 52428 Juelich (DE); Link, Thomas, 82377 Penzberg (DE); Noll, Thomas, 33615 Bielefeld (DE); Skopnik, Kerstin, 51467 Bergisch-Gladbach (DE); Wandrey, Christian, 52428 Juelich (DE)
(74) Vertreter: Burger, Alexander

(56) Entgegenhaltungen:
- WO-A1-98/41611
- CRUZ H J ET AL: "Metabolically optimised BHK cell fed-batch cultures", JOURNAL OF BIOTECHNOLOGY, ELSEVIER SCIENCE PUBLISHERS, AMSTERDAM, NL, vol. 80, no. 2, 23 June 2000 (2000-06-23), pages 109-118, XP004213500, ISSN: 0168-1656, DOI: 10.1016/S0168-1656(00)00254-6
- LJUNGGREN J ET AL: "CATABOLIC CONTROL OF HYBRIDOMA CELLS BY GLUCOSE AND GLUTAMINE LIMITED FED BATCH CULTURES", BIOTECHNOLOGY AND BIOENGINEERING, WILEY & SONS, HOBOKEN, NJ, US, vol. 44, no. 7, 20 September 1994 (1994-09-20), pages 808-818, XP002007048, ISSN: 0006-3592, DOI: 10.1002/BIT.260440706

## Beschreibung

Die Erfindung betrifft ein Verfahren zur Kultivierung von Zellen zur Produktion von Substanzen nach dem Oberbegriff des Anspruchs 1.

Bei der Produktion von Substanzen, insbesondere Proteinen, werden Zellkulturen in fermentativen Prozessen eingesetzt. Es können dabei Prozesse unterschieden werden, bei denen die Zellkulturen genetisch unverändert sind und eigene Stoffwechselprodukte bilden und bei denen die Organismen genetisch so modifiziert sind, daß sie entweder eigene Substanzen, beispielsweise Proteine, vermehrt, oder fremde Substanzen beispielsweise Proteine produzieren. Die die Substanzen produzierenden Organismen werden dabei mit einem Nährmedium versorgt, welches das Überleben der Organismen garantiert und die Produktion der gewünschten Zielverbindung ermöglicht. Für diese Zwecke sind eine Vielzahl von Kulturmedien bekannt, die eine Fermentation ermöglichen. Einer der wichtigsten Bestandteile der Kulturmedien ist die Glukose. Nach dem Stand der Technik ist man regelmäßig bemüht in einem Fermentationsansatz eine Mindestkonzentration an Glukose aufrecht zu erhalten, um die Ausbeute an Zielverbindung zu optimieren. Die japanische Patentanmeldung 001 101 882 A offenbart ein Kultivierungsverfahren für Säugetierzellen bei dem eine Mindestkonzentration von 0,2 mmol/l an Glukose aufrechterhalten wird. Die US 544 39 68 offenbart ein Kultivierungsverfahren, bei dem eine Glukoselimitierung erfolgt. Das Verfahren führt jedoch nicht zu einer höheren spezifischen Produktionsrate der Zellen gegenüber der nicht limitierenden Fütterung. Cruz, H.J., et al. (J. Biotechnol. 80 (2000) 109-118) beschreiben BHK Zellen, die metabolisch modifiziert und optimiert worden sind, zur Verwendung in fed-batch Zufütterungskulturen. Ljunggren, J., et al. (Biotechnol. Bioeng. 44 (1994) 808-818) beschreiben die Kontrolle des Katabolismus von Hybridoma-Zellen durch glukose- und glutamin-limitierten batch Kulturen.

Es ist die Aufgabe der Erfindung ein Verfahren zur Kultivierung von Zellen zu schaffen mit dem die Produktivität einer einzelnen Zelle an Produkt gesteigert wird und bei dem hohe Zelldichten ermöglicht werden. Es soll eine hohe Raum-/ZeitAusbeute an Produkt ermöglicht werden.

Das Verfahren soll besonders einfach in der Durchführung, mit minimalem Meß- und Regelaufwand verbunden, und besonders wirtschaftlich sein.

Das erfindungsgemäße Verfahren wird in den Ansprüchen beschrieben, wobei Anspruch 1 folgenden Wortlaut hat:
Verfahren zur Kultivierung von Fusionsproteinen oder Antikörper produzierenden rekombinanten CHO Zellen in einem glukosehaltigen Medium,
wobei die Kultivierung der Zelllinie unter Zufuhr eines Nährmediums erfolgt, so dass die Menge zugefütterter Glukose 50 % bis 35 % dessen ist, was die im System bei nicht glukoselimitierter Prozessführung zu erwartende Lebendzelldichte maximal verbrauchen kann,
wobei das Verfahren als Fed-Batch betrieben wird,
wobei die spezifische Produktivität der Zelle im Vergleich zu einer nicht glukoselimitierten Prozessführung erhöht ist.

Ausgehend vom Oberbegriff des Anspruchs 1 wird die Aufgabe überraschenderweise dadurch gelöst, daß die Kultivierung einer, Fusionsproteinen oder Antikörper produzierenden rekombinanten, CHO-Zellinie unter Zufuhr eines Nährmediums in der Weise erfolgt, daß sich in der Kulturlösung eine Glukoselimitierung einstellt. Der Grad der Glukoselimitierung kann definiert werden, als das Verhältnis der beobachteten spezifischen Glukoseverbrauchsrate zur maximal für diese Zellen bekannte spezifische Glukoseverbrauchsrate. Der Grad der Glukoselimitierung DGL = qGlc/qGlcₘₐₓ (qGlc = momentane beobachtete, spezifische Glukoseverbrauchsrate; qGlcₘₐₓ = maximal für diese Zellen bekannten spezifischen Glukoseverbrauchsrate). DGL liegt in den Grenzen zwischen DGL_{Erhaltung} und 1, wobei DGL_{Erhaltung} völlige Wachstumslimitierung bedeutet und 1 bedeutet keinerlei Limitierung bzw. völliger Glukoseüberschuß.

Zusammen mit der Glukoselimitierung erfolgt ein kontinuierlicher Rückgang der Restglukosekonzentration auf eine stationäre Konzentration in der Kulturlösung, die größer 0 mmol/l, jedoch kleiner als 1 mmol/l, bevorzugt kleiner als 0,5 mmol/l ist. Es ist zu beobachten, daß mit Sinken des DGL ein weiterer Anstieg der Lebendzelldichte im Kulturgefäß erfolgen kann. Mit zunehmender Glukoselimitierung konvergiert die Zelldichte dann gegen einen Maximalwert. Daraus resultiert, daß der Grad der Glukoselimitierung gegen einen minimalen Wert konvergiert, dabei ist der DGL erfindungsgemäß größer oder gleich dem DGL, welcher zur Erhaltung der Zelle führt, (maintenance Stoffwechsel)
DGL_{Erhaltung} = qGlc_{Erhaltung}/qGlc_{max.} (qGlc_{Erhaltung}= bei reinem Erhaltungsstoffwechsel beobachtete spezifische Glukoseverbrauchsrate; qGlcₘₐₓ = maximal für diese Zellen bekannten spezifischen Glukoseverbrauchsrate), und kleiner 0,5, vorzugsweise kleiner 0,4.

Charakteristisch ist jedoch, daß mit der Abnahme der Glukosekonzentration keine Abnahme der Zellkonzentration in der Lösung erfolgt. Mit zunehmender Glukoselimitierung, also bei sinkendem DGL-Wert, erhöht sich die spezifische Produktivität einer Zelle. Da die Lebendzelldichte im Kulturgefäß nicht absinkt, führt dies zu einer Erhöhung der Raum-Zeit Ausbeute. Mit Eintreten der Glukoselimitierung geht phänomenologisch eine Erniedrigung der spezifischen Laktatbildungsrate einher. Die Laktatbildungsrate konvergiert gegen einen Minimalwert. Dies führt dazu, dass die Restlaktatkonzentration im Kulturgefäß absinkt, maximal gegen 0 geht. Mit der Glukoselimitierung kommt es also zu einer Umstellung des Zellmetabolismus. Wichtig ist dabei, daß es vor Einsetzen der Glukoselimitierung zu keiner weiteren Limitierung durch andere Substrate kommt. Daher muß das Wachstumsmedium so beschaffen sein, daß Glukose zuerst limitiert ist.

Mit dem erfindungsgemäßen Verfahren wird die Raum-/ZeitAusbeute bei gegebener Zelldichte erhöht. Durch das erfindungsgemäße Verfahren wird die pro Zelle zur Verfügung stehende Menge an Glukose derart vermindert, daß die Glukose überwiegend in den Erhaltungsstoffwechsel und verbunden damit das Produkt und weniger in Zellwachstum eingeht. Das erfindungsgemäße Verfahren bedarf dabei nicht einer Regelung der Glukosezufütterung, so daß das Verfahren besonders einfach ist, da auf eine aufwendige Glukoseregelung verzichtet werden kann. Dadurch, daß ein geringer Medienzufluss nötig ist, werden Kosten für Glukose gespart, da weniger Glukose benötigt wird. Zudem wird eine sehr hohe Produktkonzentration erreicht. Dies kann zur Senkung der Aufarbeitungskosten führen. Das erfindungsgemäße Verfahren ermöglicht insbesondere die Steigerung der Produktion von Proteinen, ohne daß eine Zellinie für die Umsetzung des erfindungsgemäßen Verfahrens zusätzlich genetisch verändert werden muss. Die Erhöhung des Produkttiters ermöglicht die Produktion einer gewünschten Menge an Produkten in einem kleineren Kultivierungsvolumen, was zu geringeren Investitionskosten führt.

Das erfindungsgemäße Verfahren kann mit folgenden Verfahrensschritten durchgeführt werden:
Vorzugsweise sollte das Kultursystem hohe Zelldichten ermöglichen. Vorzugsweise wird eine Zellrückhaltung realisiert, damit die Zelldichte bei Auftreten der Glukoselimitierung nicht absinken kann. Dies führt dazu, dass bei steigender Lebendzelldichte und gleichbleibender Glukosezufütterung, der DGL weiter verringert wird. Die hohe Zelldichte ermöglicht ein Absinken des DGL unter einen Wert von 0,4.

Um eine Verringerung des DGL zu erreichen, kann die Fütterungsstrategie mit Glukose demnach wie folgt erfolgen: Die Menge an zugefütterter Glukose wird nicht mit zunehmender Lebendzelldichte erhöht, um eine Glukoselimitierung zu vermeiden. Vielmehr wird die Menge an zugefütterter Glukose während des Prozesses von Beginn an konstant gehalten. Dabei sollte die Menge an zugefütterter Glukose so gewählt sein, dass der DGL die erforderlichen Werte unterschreitet, nämlich DGL kleiner ≤ 0,5, vorzugsweise ≤ 0,4. Daraus resultiert, daß die Menge zugefütterter Glukose vorzugsweise nicht grösser als 50 % und nicht weniger als 35 % dessen ist, was die im System bei herkömmlicher, nicht glukoselimitierter Prozeßführung zu erwartende Lebendzellzahl maximal verbrauchen kann. Nach Umstellung des Zellmetabolismus (Laktatstoffwechel und Produktivität) kann die Menge zugefütterter Glukose langsam erhöht werden, sollte dabei jedoch nicht einen DGL von größer als 0,5, vorzugsweise größer als 0,4 ermöglichen. Dies führt zu einer weiteren Erhöhung der Lebendzelldichte bei gleichbleibend hoher Produktivität und damit erhöhter Raum-/Zeit-Ausbeute. Die Menge zugeführter Glukose kann in einem kontinuierlichen Prozeß durch die Medienzuflußrate und die Glukosekonzentration im Zufütterungsmedium beeinflußt werden. Maßgeblich ist, dass der Massenfluss an zugeführter Glukose während des Prozesses nicht oder nur in solchem Maße erhöht wird, daß der DGL einen Wert von kleiner 0,5, vorzugsweise kleiner 0,4 erreicht oder unterschreitet und dieser dann nicht mehr überschritten wird.

Vorteilhafte Weiterbildungen der Erfindung sind in den Unteransprüchen angegeben.

Im Folgenden soll die Erfindung in ihren Einzelheiten dargestellt werden.

Die Figuren zeigen beispielhafte Versuchsergebnisse.

Es zeigt:
- Fig.1:: Zunahme der vitalen Zellzahl [ml⁻¹] und Darstellung der Mediendurchflußrate [h⁻¹] gegen die Prozesszeit [h] für die Produktion von MUC1-IgG2a aus CHO MUC1/IgG2a PH3744/25 Zellen im Perfusionsreaktor.
- Fig.2:: Spezifische Produktivität an MUC1-IgG2a [µg/h*E9 Zellen)] und DGL gegen die Prozesszeit im Perfusionsreaktor.
- Fig.3:: Zunahme der vitalen Zellzahl [ml⁻¹] und mM Restglukose, aufgetragen gegen die Prozesszeit [h] für die Produktion von MUC1-IgG2a aus CHO MUC1/IgG2a PH3744/25 Zellen im Perfusionsreaktor.
- Fig.4:: Glukose- und Laktatkonzentration sowie Konzentration von Glukose im Medienzulauf [mmol/l], aufgetragen gegen die Prozesszeit [h] für die Produktion von MUC1-IgG2a aus CHO MUC1/IgG2a PH3744/25 Zellen im Perfusionsreaktor.
- Fig.5:: Zunahme der Konzentration an MUC1-IgG2a [µg/ml] und qMUC1-IgG2a [µg/h*E9 Zellen)] gegen die Zeit [h] für die Produktion von MUC1-IgG2a aus CHO MUC1/IgG2a PH3744/25 Zellen im Perfusionsreaktor.
- Fig.6:: Zunahme der vitalen Zellzahl [ml⁻¹] und Darstellung der Mediendurchflußrate [h⁻¹] gegen die Prozesszeit [h] für die Produktion von MUC2-GFP-C-term aus CHO MUC2-GFP-C-term Zellen im Perfusionsreaktor.
- Fig.7:: Spezifische Produktivität an MUC2-GFP-C-term [nmol/h*E9 Zellen)] und DGL gegen die Prozesszeit im Perfusionsreaktor.
- Fig.8:: Zunahme der vitalen Zellzahl [ml⁻¹] und Restglukose [mM], aufgetragen gegen die Prozesszeit [h] für die Produktion von MUC2-GFP-C-term aus CHO MUC2-GFP-C-term Zellen im Perfusionsreaktor
- Fig.9:: Glukose- und Laktatkonzentration sowie Konzentration von Glukose im Medienzulauf [mmol/l], aufgetragen gegen die Prozesszeit [h] für die Produktion von MUC2-GFP-C-term aus CHO MUC2-GFP-C-term Zellen im Perfusionsreaktor
- Fig.10: Zunahme der Konzentration an MUC2-GFP-C-term [nM] und qMUC2-GFP-C-term [nmol/h*E9 Zellen)] gegen die Zeit [h] für die Produktion von MUC2-GFP-C-term aus CHO MUC2-GFP-C-term Zellen im Perfusionsreaktor.

Weiterhin zeigt Tabelle 1 die Versuchsdaten aus der Anwendung des erfindungsgemäßen Verfahrens mit der CHO MUC1/IgG2a PH 3744/25 Zelle.

In Tabelle 2 sind die Versuchsdaten aus der Anwendung des erfindungsgemäßen Verfahrens mit der CHO MUC2-GFP-C-term Zelle dargestellt.

Die erfindungsgemäße Verfahrensweise kann mit verschiedenen Produktionszellinien durchgeführt werden. Die Zellinien können als genetisch modifizierte, rekombinante Zellen eingesetzt werden. Die genetische Modifikation kann beispielsweise durch Insertion von zusätzlichen Genen des gleichen Organismus oder eines anderen Organismus in die DNA, oder einen Vektor erfolgen, oder in der Verstärkung der Aktivität bzw. Expression eines Gens durch Einbringen eines wirksameren Promotors, zum Beispiel aus CMV. Die Gene können für verschiedene Proteine kodieren, beispielsweise für Proteine, wie Fusionsproteine oder für Antikörper.

Folgende Zellinien können beispielhaft genannt werden: Säugerzellen, wie CHO Zellinien, wie zum Beispiel CHO-K1, BHK, wie BHK-21, Hybridoma, NS/0, andere Myelomazellen und Insektenzellen oder andere höhere Zellen. Besonders bevorzugt ist die Verwendung von Zellen, die nicht vorzugsweise wachstumsgekoppelt produzieren.

Eine rekombinante CHO Zellinie deren Produktivität mit der erfindungsgemäßen Verfahrensweise gesteigert werden kann ist die Zellinie CHO MUC1/IgG2a, PH 3744/25, mit der das Glykoprotein MUCl-IgG2a sekretiert werden kann. Eine weitere CHO Zellinie, nämlich CHO MUC2-GFP-C-term, ist befähigt ein Fusionsprotein MUC2-GFP-C-term gesteigert zu sekretieren, wenn sie der erfindungsgemäßen Verfahrensweise unterzogen wird.

Als Kulturmedium kann prinzipiell jedes glukosehaltige Medium eingesetzt werden, welches bezüglich anderer Komponenten nicht limitierend ist. Beispielhaft kann ProCHO4-CDM genannt werden. Es können auch Medien basierend auf bekannten Rezepturen, wie zum Beispiel IMDM, DMEM oder Ham's F12 eingesetzt werden, die so auf die erfindungsgemäße Verfahrensweise optimiert wurden, daß lediglich Glukoselimitierung auftritt. Dies kann zum Beispiel dadurch erreicht werden, dass andere Komponenten im Verhältnis zur Glukose höher konzentriert werden. Generell ist es auch möglich die Glukose separat vom Medium zu zu dosieren.

Der PH Bereich liegt vorzugsweise zwischen 6,7 - 7,7, besonders bevorzugt zwischen 7 - 7,3. Jedoch sind auch andere pH-Bereiche denkbar.

Der Temperaturbereich liegt vorzugsweise zwischen 35 °C - 38,5 °C, besonders bevorzugt bei 37 °C für CHO MUC1-IgG2a. Es sind aber auch andere Temperaturbereiche denkbar, wie beispielsweise < 35 °C, bei denen es nicht zur irreversiblen Zerstörung des Produkts kommt.

Mit dem hierin beschriebenen Kultivierungsverfahren können Substanzen, wie Glykoproteine, Fusionsproteine, Antikörper, Proteine im Allgemeinen produziert werden, von denen beispielhaft MUC1-IgG2a, MUC2-GFP-C-term, EPO, Interferone, Cytokine, Wachstumsfaktoren, Hormone, PA, Immunglobuline oder Fragmente von Immunglobulinen, genannt werden können.

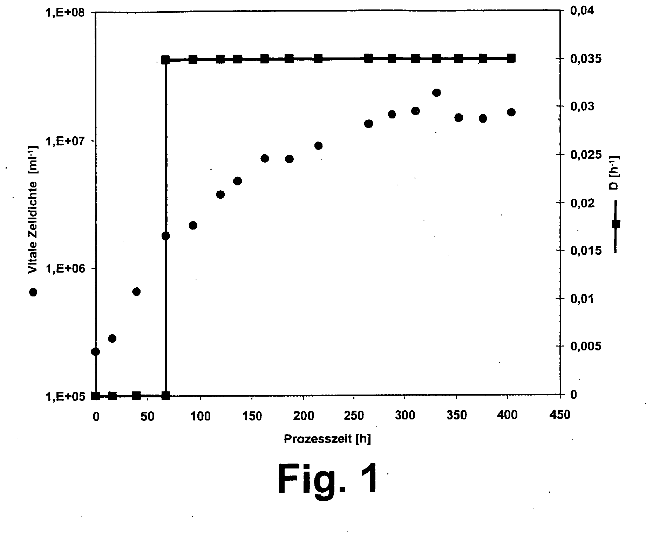

Figur 1 zeigt den Verlauf der Lebendzelldichte (cv) an CHO/MUC1-IgG2a Zellen und der Mediendurchflußrate (D) gegen die Prozesszeit (h) im Perfusionsreaktor. In ihr ist:
▪ Die Mediendurchflußrate (1/h) und
• die Lebendzelldichte (1/ml).

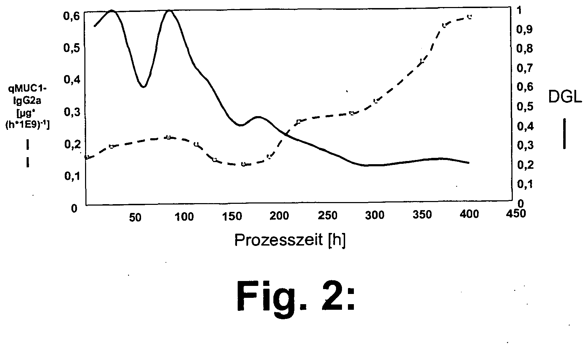

Figur 2 zeigt die spezifische Produktivität an MUC1-IgG2a (qMUC1-IgG2a) und DGL gegen die Prozesszeit im Perfusionsreaktor. In ihr ist:
- - - Die spezifische Produktivität (µg/hE9 Zellen),
- DGL(degree of glucose limitation)

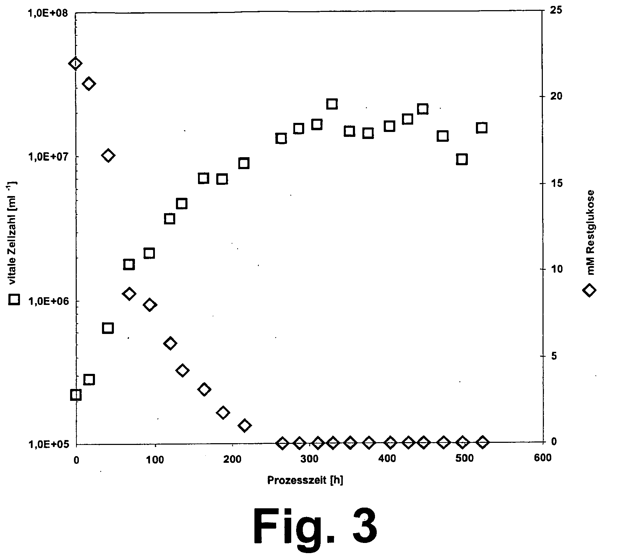

Figur 3 zeigt eine Graphik, in der auf der linken Seite die vitale Zellzahl [ml⁻¹] und auf der rechten Seite die Konzentration der Restglukose [mM] gegen die Prozesszeit [h] für die Produktion von MUC1-IgG2 in CHO MUC/IgG2a PH3744/25 aufgetragen ist. In ihr ist:
□ vitale Zellzahl und
◇ Glukose.

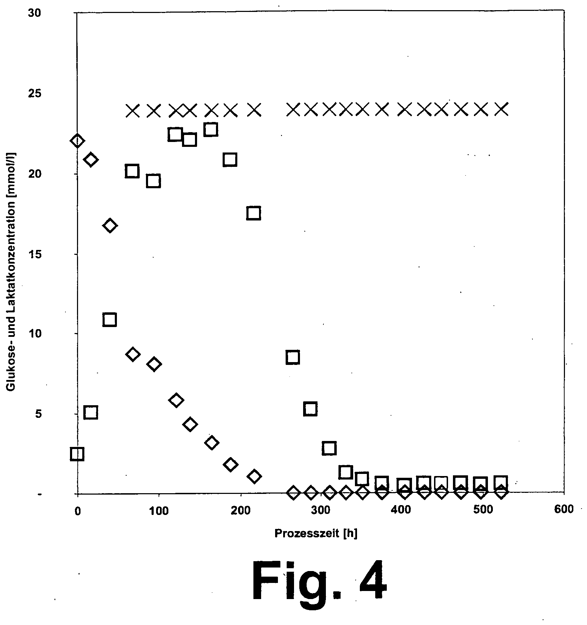

In Figur 4 ist die Glukose- und Laktatkonzentration sowie die Glukosekonzentration im Medienzulauf [mmol/1] gegen die Prozesszeit [h] aufgetragen. In ihr sind die Kurven mit
□ Laktatkonzentrationskurven und
◇ Glukosekonzentrationskurven.
x 23,9 mmol/l Konzentration an Glukose im Medienzulauf (Durchflussrate von D = 0,035 h⁻¹).

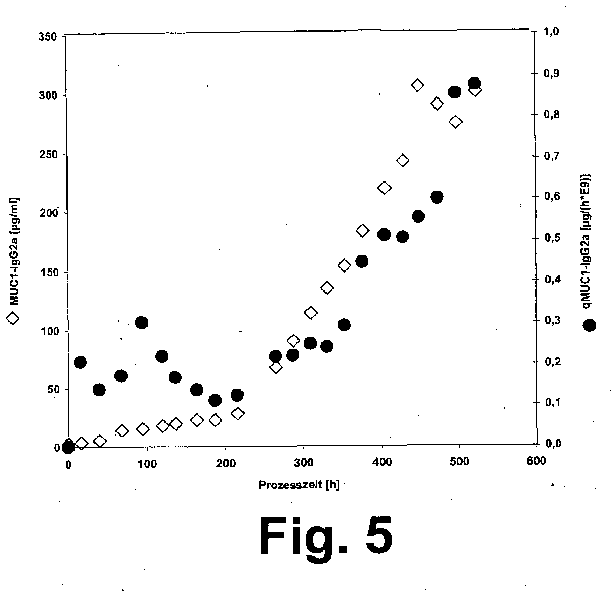

In Figur 5 ist die Konzentration von MUC1-IgG2a [µg/ml] auf der linken Seite sowie qMUC1-IgG2a [µg/(h^{∗}E9 Zellen)] auf der rechten Seite der Graphik gegen die Zeit [h] aufgetragen. In ihr sind:
• q spezifische Produktivität an MUC1-IgG2a (µg/hE9 Zellen) und
◇ Konzentration an MUC1-IgG2a (mg/l).

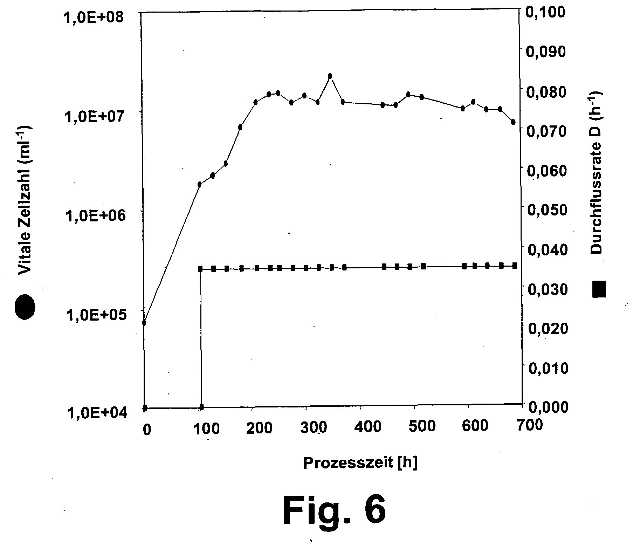

Figur 6 zeigt den Verlauf der Lebendzelldichte (cv) an CHO/MUC2-GFP Zellen und der Mediendurchflußrate (D) gegen die Prozesszeit (h) im Perfusionsreaktor. In ihr ist:
▪ Die Mediendurchflußrate (1/h) und
• die Lebendzelldichte (1/ml).

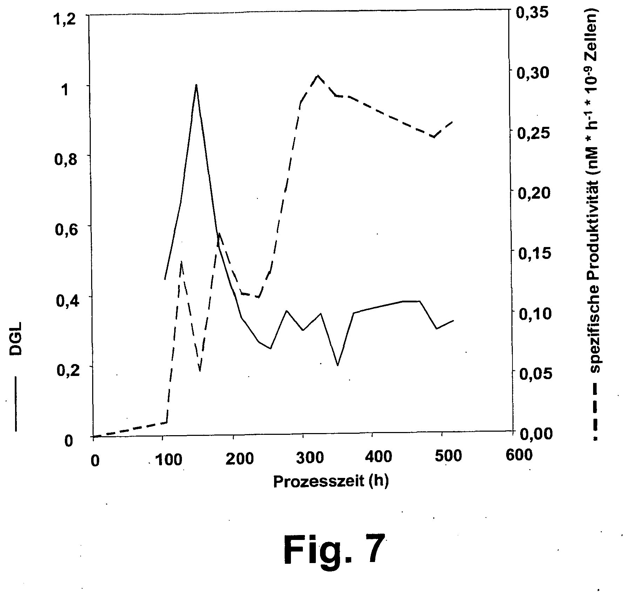

Figur 7 zeigt die spezifische Produktivität an MUC2-GFP-C-term (qMUC2-GFP-C-term) und DGL gegen die Prozeßzeit im Perfusionsreaktor. In ihr ist:
- - - Die spezifische Produktivität (nmol/hE9 Zellen),
- DGL(degree of glucose limitation)

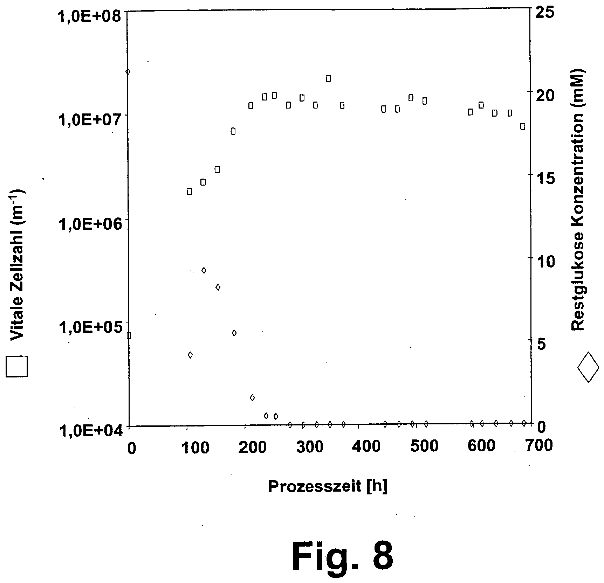

Figur 8 zeigt eine Graphik, in der auf der linken Seite die vitale Zellzahl [ml⁻¹] und auf der rechten Seite die Konzentration der Restglukose [mM] gegen die Prozesszeit [h] für die Produktion von MUC2-GFP-C-term in CHO MUC/IgG2a PH3744/25 aufgetragen ist. In ihr ist:
□ vitale Zellzahl und
◇ Glukose.

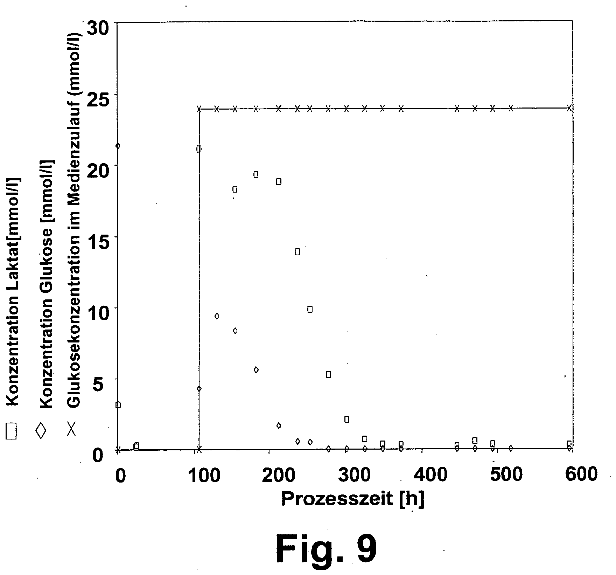

In Figur 9 ist die Glukose- und Laktatkonzentration sowie die Glukosekonzentration im Medienzulauf [mmol/l] gegen die Prozesszeit [h] aufgetragen. In ihr sind die Kurven mit
□ Laktatkonzentrationskurven und
◇ Glukosekonzentrationskurven.
x 23,9 mmol/l Konzentration an Glukose im Medienzulauf (Durchflußrate von D = 0,035 h⁻¹).

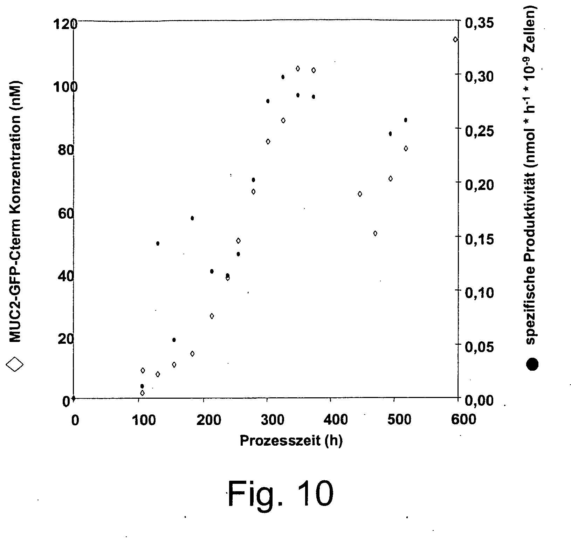

In Figur 10 ist die Konzentration von MUC2-GFP-C-term [nM] auf der linken Seite sowie qMUC2-GFP-C-term [nmol/(h^{∗}E9 Zellen)] auf der rechten Seite der Graphik gegen die Zeit [h] aufgetragen. In ihr sind:
• q spezifische Produktivität an MUC2-GFP-C-term (nmol/hE9 Zellen) und
◇ Konzentration an MUC2-GFP-C-term (nM).

Figur 1 zeigt die Verfahrensweise beispielhaft betreffend die Glukosezufütterung. In kontinuierlicher Perfusionskultur wird eine konstante Menge Glukose zugefüttert. Im gezeigten Beispiel wird dies durch eine konstante Mediendurchflußrate erreicht, wobei die Glukosekonzentration im Medienzulauf konstant ist. Die Mediendurchflußrate wird nicht mit zunehmender Lebendzelldichte erhöht. Der Prozess wurde als Batch begonnen, bevor die kontinuierliche Verfahrensweise begann.

Figur 2 zeigt, daß bei dieser Verfahrensweise der DGL im Verlaufe des Prozesses sinkt, und schließlich einen Wert unter 0,4 erreicht. Während dies geschieht, steigt die spezifische Produktivität an und erreicht schließlich einen Wert der um das 4-fache höher ist, als der vor Unterschreiten des DGL-Wertes von 0,4.

Aus Figur 3 wird ersichtlich, dass bei dem erfindungsgemäßen Verfahren die Lebendzelldichte gegen einen Maximalwert läuft, der dann gehalten werden kann, während die Restglukosekonzentration im Verlauf gegen null geht. Dies tritt ein, obwohl Glukose zugeführt wird. Während des Absinkens der Restglukosekonzentration, beginnt die spezifische Glukoseaufnahmerate der Organismen zu sinken. Während dessen kann die Lebendzellzahl noch ansteigen. Parallel zum Rückgang der spezifischen Glukoseaufnahmerate sinkt auch die spezifische Laktatbildungsrate, was zunächst zu einem verlangsamten Anstieg, dann zu einem Abfall der Laktatkonzentration im Kulturgefäß führt. Schließlich läuft die Laktatkonzentration im Kulturgefäß gegen null, wie aus Figur 4 zu entnehmen ist. Es liegt also eine deutliche Umstellung des Zellmetabolismus vor. Wie Figur 5 zu entnehmen ist, findet verbunden mit der Umstellung des Zellmetabolismus ein Anstieg der spezifischen Produktivität auf etwa das 4-fache gegenüber dem Zeitpunkt vor der Umstellung des Zellmetabolismus statt. Der Anstieg der spezifischen Produktivität bei mindestens gleichbleibenden, oder sogar noch ansteigenden Zelldichten während der beschriebenen Phase führt schließlich zu einem markanten Anstieg des Produkttiters im Kulturüberstand, wie Figur 5 zu entnehmen ist, und damit zu einer erhöhten Raum-/Zeit-Ausbeute.

Tabelle 1 zeigt Daten zur Fermentation von MUC1-IgG2a.

Analog zu den Figuren 1 bis 5 beschreiben Figuren 6 bis 10 die Ergebnisse der Anwendung des erfindungsgemäßen Verfahrens mit CHO MUC2-GFP-C-term Zellen.

Tabelle 2 zeigt Daten zur Fermentation von MUC2-GFP-C-term.

Produktionstechnisch kann das erfindungsgemäße Verfahren außer nach dem eben beschriebenen Perfusionsverfahren auch als Fed-Batch (Zufütterungsverfahren) betrieben werden.

In einem Fed-Batch-Betrieb wird die Produktionskultur einmal oder wiederkehrend, bzw. chargenweise oder kontinuierlich mit glukosehaltigem Medium oder einer separaten Glukoselösung in einer Art und Weise versorgt, daß der DGL vorzugsweise den Wert von 0,5, besonders bevorzugt 0,4 unterschreitet. Möglich ist hier auch ein repetitiver Fed-Batch.

Sowohl in der perfusiven Verfahrensweise als auch im Fed-Batch kann der Prozeß in allen allgemein bekannten Verfahrensweisen begonnen werden. So kann vor Beginn der erfindungsgemäßen Verfahrensweise die Kultur als Batch, Fed-Batch oder in kontinuierlicher Verfahrensweise mit oder auch ohne Zellrückhaltung betrieben werden.

**Tabelle 1: Daten zur Fermentation von MUC1-IgG2a**

| Prozesszeit | cv | D | Glukose Feed | Glukose | Laktat | MUC1-IgG2a | qMUC1-IgG2a | DGL |
|---|---|---|---|---|---|---|---|---|
| h | 1/ml | 1/h | mmol/l | mmol/l | mmol/l | µg/ml | µg/(h*E9) | |
| 0 | 2,23E+05 | 0 | 0 | 22,07 | 2,5 | 2,62 | | |
| 16,63 | 2,83E+05 | 0 | 0 | 20,89 | 5,1 | 3,59 | 0,21 | 0,92 |
| 40,52 | 6,48E+05 | 0 | 0 | 16,75 | 10,84 | 5,77 | 0,14 | 0,99 |
| 68 | 1,78E+06 | 0 | 0 | 8,74 | 20,1 | 14,21 | 0,17 | 0,61 |
| 94 | 2,14E+06 | 0,035 | 23,89 | 8,08 | 19,48 | 15,49 | 0,30 | 1,00 |
| 120 | 3,70E+06 | 0,035 | 23,89 | 5,84 | 22,35 | 18,02 | 0,22 | 0,72 |
| 136,5 | 4,68E+06 | 0,035 | 23,89 | 4,30 | 22,02 | 19,95 | 0,17 | 0,62 |
| 163,5 | 7,02E+06 | 0,035 | 23,89 | 3,17 | 22,66 | 22,67 | 0,14 | 0,40 |
| 187,5 | 6,96E+06 | 0,035 | 23,89 | 1,79 | 20,77 | 22,44 | 0,11 | 0,44 |
| 215,5 | 8,85E+06 | 0,035 | 23,89 | 1,04 | 17,46 | 28,24 | 0,13 | 0,35 |
| 264,75 | 1,30E+07 | 0,035 | 23,89 | - | 8,45 | 67,03 | 0,22 | 0,24 |
| 287 | 1,54E+07 | 0,035 | 23,89 | - | 5,25 | 89,42 | 0,22 | 0,20 |
| 310 | 1,64E+07 | 0,035 | 23,89 | - | 2,77 | 113,28 | 0,25 | 0,19 |
| 331 | 2,27E+07 | 0,035 | 23,89 | - | 1,24 | 133,80 | 0,24 | 0,14 |
| 352,4 | 1,45E+07 | 0,035 | 23,89 | - | 0,82 | 152,87 | 0,29 | 0,21 |
| 376,3 | 1,42E+07 | 0,035 | 23,89 | - | 0,53 | 182,52 | 0,45 | 0,22 |
| 404,4 | 1,58E+07 | 0,035 | 23,89 | - | 0,44 | 218,51 | 0,51 | 0,20 |
| 428 | 1,78E+07 | 0,035 | 23,89 | - | 0,58 | 241,75 | 0,50 | 0,17 |
| 448,4 | 2,08E+07 | 0,035 | 23,89 | - | 0,55 | 305,39 | 0,55 | 0,15 |
| 473,63 | 1,35E+07 | 0,035 | 23,89 | - | 0,55 | 290,52 | 0,60 | 0,23 |
| 496,8 | 9,30E+06 | 0,035 | 23,89 | - | 0,51 | 274,94 | 0,85 | 0,33 |
| 521,82 | 1,53E+07 | 0,035 | 23,89 | - | 0,56 | 301,12 | 0,87 | 0,20 |

**Tabelle 2: Daten zur Fermentation von MUC2-GFP-Cterm**

| Prozeßzeit | Vitale Zellzahl | D | Glukose Feed | Glukose | Laktat | MUC2-GFP-Cterm | qProdukt | DGL |
|---|---|---|---|---|---|---|---|---|
| h | 1/ml | 1/h | mmol/l | mmol/l | mmol/l | nM | nmol/(h*E9) | |
| 0,5 | 7,50E+04 | 0 | 0 | 21,37 | 3,12 | 0,00 | | |
| 106 | 1,80E+06 | 0 | 0 | 4,25 | 21,1 | 1,66 | 0,01 | 0,44 |
| 106,01 | | 0,035 | 23,89 | | | 8,92 | | |
| 130 | 2,20E+06 | 0,035 | 23,89 | 9,36 | | 7,71 | 0,14 | 0,66 |
| 154 | 2,90E+06 | 0,035 | 23,89 | 8,32 | 18,23 | 10,72 | 0,05 | 1,00 |
| 182,38 | 6,83E+06 | 0,035 | 23,89 | 5,58 | 19,28 | 14,08 | 0,17 | 0,53 |
| 212,9 | 1,19E+07 | 0,035 | 23,89 | 1,65 | 18,78 | 26,15 | 0,12 | 0,33 |
| 237,2 | 1,44E+07 | 0,035 | 23,89 | 0,54 | 13,84 | 38,37 | 0,11 | 0,26 |
| 254 | 1,48E+07 | 0,035 | 23,89 | 0,52 | 9,81 | 50,08 | 0,13 | 0,24 |
| 278 | 1,20E+07 | 0,035 | 23,89 | - | 5,19 | 65,63 | 0,20 | 0,35 |
| 302 | 1,40E+07 | 0,035 | 23,89 | - | 2,05 | 81,53 | 0,27 | 0,29 |
| 326 | 1,20E+07 | 0,035 | 23,89 | - | 0,7 | 88,03 | 0,30 | 0,34 |
| 349,9 | 2,16E+07 | 0,035 | 23,89 | - | 0,33 | 104,60 | 0,28 | 0,19 |
| 374 | 1,20E+07 | 0,035 | 23,89 | - | 0,26 | 104,03 | 0,28 | 0,34 |
| | | 0,035 | 23,89 | - | | 84,47 | | |
| | | 0,035 | 23,89 | - | | 75,16 | | |
| 446 | 1,10E+07 | 0,035 | 23,89 | - | 0,19 | 64,81 | | 0,37 |
| 470 | 1,10E+07 | 0,035 | 23,89 | - | 0,53 | 52,36 | | 0,37 |
| 494 | 1,40E+07 | 0,035 | 23,89 | - | 0,32 | 69,63 | 0,24 | 0,29 |
| 518 | 1,30E+07 | 0,035 | 23,89 | - | | 79,34 | 0,26 | 0,32 |
| | | 0,035 | 23,89 | - | | 93,94 | | |
| | | 0,035 | 23,89 | - | 0,35 | 104,57 | | |
| 595,8 | 1,01E+07 | 0,035 | 23,89 | - | 0,25 | 113,89 | | |

## Patentansprüche

1. Verfahren zur Kultivierung von Fusionsproteinen oder Antikörper produzierenden rekombinanten CHO Zellen in einem glukosehaltigen Medium, wobei die Kultivierung der Zelllinie unter Zufuhr eines Nährmediums erfolgt, so dass die Menge zugefütterter Glukose 50 % bis 35 % dessen ist, was die im System bei nicht glukoselimitierter Prozessführung zu erwartende Lebendzelldichte maximal verbrauchen kann,
wobei das Verfahren als Fed-Batch betrieben wird,
wobei die spezifische Produktivität der Zelle im Vergleich zu einer nicht glukoselimitierten Prozessführung erhöht ist.

2. Verfahren nach Anspruch 1, **dadurch gekennzeichnet, dass** die Kultivierung der Zellen unter Glukoselimitierung erfolgt, wobei das Verhältnis der beobachteten spezifischen Glukoseverbrauchsrate zur maximal für diese Zellen bekannten spezifischen Glukoseverbrauchsrate kleiner als 0,5 ist.

3. Verfahren nach Anspruch 2, **dadurch gekennzeichnet, dass** das Verhältnis kleiner als 0,3 ist.

4. Verfahren nach einem der Ansprüche 2 oder 3, **dadurch gekennzeichnet, dass** das Verhältnis während der Kultivierung sinkt.

5. Verfahren nach einem der vorhergehenden Ansprüche, **dadurch gekennzeichnet, dass** die Menge an zugefütterter Glukose von beginn an konstant gehalten wird.

6. Verfahren nach einem der vorhergehenden Ansprüche, **dadurch gekennzeichnet, dass** die Säugerzelle eine CHO-K1 Zelle ist.

7. Verfahren nach einem der vorhergehenden Ansprüche, **dadurch gekennzeichnet, dass** ein Antikörper produziert wird.

8. Verfahren nach einem der Ansprüche 5 bis 7, **dadurch gekennzeichnet, dass** die Kultivierung als Batch begonnen worden ist.

## Claims

1. A method for cultivating fusion protein- or antibody-producing recombinant CHO cells in a glucose-containing medium,
wherein the cultivation of the cell line is carried out with the addition of a nutrient medium such that the amount of supplemented glucose is 50% to 35% of that which can maximally be consumed by the living cell density to be expected in the system in the case of a non-glucose-limited process,
wherein the method is operated as a fed-batch method,
wherein the specific productivity of the cell is increased compared to a non-glucose-limited process.

2. The method according to claim 1, **characterised in that** the cultivation of the cells is carried out under glucose limitation, wherein the ratio of the observed specific glucose consumption rate to the maximum specific glucose consumption rate known for these cells is less than 0.5.

3. The method according to claim 2, **characterised in that** the ratio is less than 0.3.

4. The method according to claim 2 or claim 3, **characterised in that** the ratio decreases during the cultivation.

5. The method according to one of the foregoing claims, **characterised in that** the amount of supplemented glucose is held constant from the beginning.

6. The method according to one of the foregoing claims, **characterised in that** the mammalian cell is a CHO-K1 cell.

7. The method according to any one of the foregoing claims, **characterised in that** an antibody is produced.

8. The method according to any one of claims 5 to 7, **characterised in that** the cultivation has been started as a batch.

## Revendications

1. Procédé de culture de cellules CHO recombinantes produisant de protéines de fusion ou des anticorps dans un milieu contenant du glucose,
la lignée cellulaire étant cultivée avec apport d'un milieu nutritif, de sorte que la quantité de glucose fourni représente 50 % à 35 % de ce que peut consommer au maximum la densité de cellules vivantes à laquelle on peut s'attendre dans le système dans le cas d'un procédé sans limitation du glucose,
le procédé étant exploité sous forme discontinue alimentée,
la productivité spécifique de la cellule étant augmentée par rapport à un procédé sans limitation du glucose.

2. Procédé selon la revendication 1, **caractérisé en ce que** la culture des cellules a lieu avec une limitation du glucose, le rapport du taux de consommation spécifique du glucose observé et du taux de consommation spécifique du glucose maximum connu pour ces cellules étant inférieur à 0,5.

3. Procédé selon la revendication 2, **caractérisé en ce que** le rapport est inférieur à 0,3.

4. Procédé selon l'une des revendications 2 ou 3, **caractérisé en ce que** le rapport diminue pendant la culture.

5. Procédé selon l'une des revendications précédentes, **caractérisé en ce que** la quantité de glucose fourni est maintenue constante dès le début.

6. Procédé selon l'une des revendications précédentes, **caractérisé en ce que** la cellule mammifère est une cellule CHO-K1.

7. Procédé selon l'une des revendications précédentes, **caractérisé en ce qu'**un anticorps est produit.

8. Procédé selon l'une des revendications 5 à 7, **caractérisé en ce que** la culture a été commencée en lot.
